# EUROPEAN PATENT APPLICATION

(11) **EP 1 737 052 A1**
(43) Date of publication of application: **27.12.2006**
(21) Application number: 05013304.0
(22) Date of filing: 20.06.2005
(51) Int. Cl.: H01L 35/00, H01L 23/38, H01L 35/30, A41D 13/005

(54) **Heat adjustment device**

(71) Applicant: Well &David Corporation, Kaohsiung City 804 (TW)
(72) Inventor: Hsieh, Ker-Chang, Kaohsiung City 804 (TW); Cheng, Chi-Cheng, Kaohsiung City 804 (TW); Ho, New-Jin, Kaohsiung City 804 (TW)
(74) Representative: Reitstötter - Kinzebach

(57) **Abstract**

The invention relates to a heat adjustment device. The heat adjustment device (10) comprises a heat conductive substrate (11) and a plurality of thermoelectric chips (12,13,14,15,16). The heat conductive substrate has a support surface. Each of the thermoelectric chips has a first surface (121) and a second surface (122), wherein the second surfaces (122) of the thermoelectric chips are fixed to the support surface (111) of the heat conductive substrate, and the thermoelectric chips are electrically connected for providing a power to the thermoelectric chips so that the second surfaces (122) of the thermoelectric chips give a temperature variation conducted to the heat conductive substrate (11). According to the heat adjustment device of the invention, after the temperature variation is conducted by the thermoelectric chips to the heat conductive substrate, a corresponding temperature variation is produced on the heat conductive substrate so that the area of the temperature variation is increased and the temperature is uniformly distributed on the heat conductive substrate. The heat adjustment device of the invention can be mounted in clothes. When the temperature of the second surfaces of the thermoelectric chips is increased and the heat is conducted to the heat conductive substrate, the clothes can produce heat to keep warm.

## Description

### BACKGROUND OF THE INVENTION

### 1. Field of the Invention

The invention relates to a heat adjustment device, particularly to a heat adjustment device utilizing thermoelectric chips.

### 2. Description of the Related Art

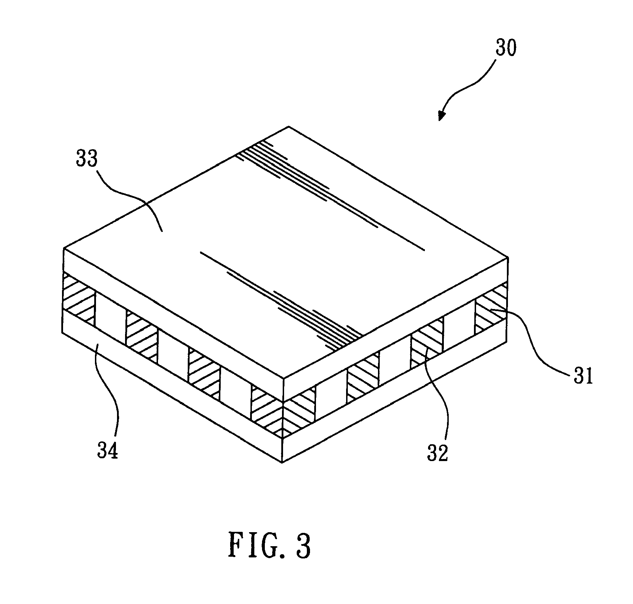

The thermoelectric chips are generally used for heat elimination of the central processing unit (CPU) or integrated circuit. Referring to figure 3, the conventional thermoelectric chip 30 is consisted of a plurality of N-type semiconductors 31 and a plurality of P-type semiconductors 32 interleaved together. The N-type semiconductors 31 and P-type semiconductors 32 are electrically connected by conductors. The thermoelectric chip 30 further comprises two ceramic substrates 33 and 34, so as to package the N-type semiconductors 31 and P-type semiconductors 32.

When the power supply is supplied to the thermoelectric chip 30, one of the two ceramic substrates 33 will produce heat from the thermoelectric chip 30, and another ceramic substrate 34 will absorb the heat to achieve a cooling effect. Therefore, the ceramic substrate 34 of the thermoelectric chip is usually used in contact with the substance to be cooled, for example the central processing unit of a computer, so as to lower the temperature of the central processing unit. Moreover, the ceramic substrate 33 which produces the heat can have the heat evacuated by contacting with a heat sink or cooling fan.

The prior art references disclose a helmet cooling system having a thermoelectric chip. The thermoelectric chip is used to remove heat from a liquid cushion, through a flexible braided wire "heat collector" located within the cushion. Heat is exhausted to ambient through an "outboard" heat sink finned aluminum radiator. However, the conventional helmet cooling system only utilizes a single thermoelectric chip, and the single thermoelectric chip can't effectively remove heat.

Therefore, it is necessary to provide a heat adjustment device so as to solve the above problem.

### SUMMARY OF THE INVENTION

The object of the invention is to provide a heat adjustment device. The heat adjustment device comprises a heat conductive substrate and a plurality of thermoelectric chips. The heat conductive substrate has a support surface. Each of the thermoelectric chips has a first surface and a second surface, wherein the second surfaces of the thermoelectric chips are fixed to the support surface of the heat conductive substrate, and the thermoelectric chips are electrically connected for providing power to the thermoelectric chips so that the second surfaces of the thermoelectric chips have a temperature variation conducted to the heat conductive substrate.

According to the heat adjustment device of the invention, after the temperature variation is conducted by the thermoelectric chips to the heat conductive substrate, a corresponding temperature variation can be produced on the heat conductive substrate so that the area of the temperature variation is increased and the temperature is uniformly distributed on the heat conductive substrate. The heat adjustment device of the invention can be mounted in clothes. When the temperature of the second surfaces of thermoelectric chips is increased and the heat is conducted to the heat conductive substrate, the clothes can produce heat to keep warm.

### BRIEF DESCRIPTION OF THE DRAWINGS

Fig. 1 is a structural schematic drawing of the heat adjustment device of the invention;
Fig. 2 shows the corresponding temperature variations of the heat conductive substrate measured for different input voltages; and
Fig. 3 is a structural schematic drawing of a conventional thermoelectric chip.
Fig. 4 shows a front elevational view of a jacket with a plurality of heat adjustment devices, according to the invention.
Fig. 5 shows a rear elevational view of a jacket with a plurality of heat adjustment devices, according to the invention.
Fig. 6 shows a packet with a heat adjustment device, according to the invention.
Fig. 7 shows a hat with a heat adjustment device, according to the invention.
Fig. 8 shows an inside view of a glove with a heat adjustment device, according to the invention.
Fig. 9 shows a structural schematic drawing of a glove with a heat adjustment device, according to the invention.
Fig. 10 shows a matress with a plurality of heat adjustment devices, according to the invention.
Fig. 11 shows a bedclothes with a plurality of heat adjustment devices, according to the invention.

### DETAILED DESCRIPTION OF THE INVENTION

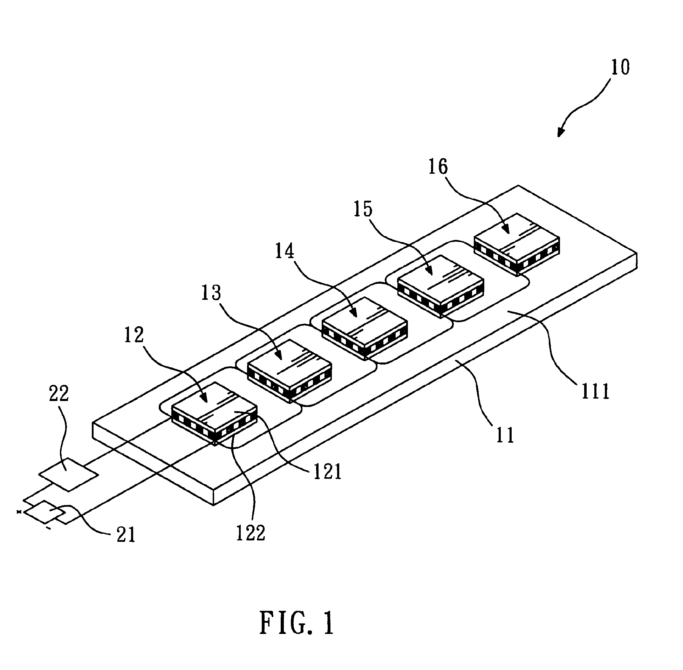

Referring to Fig. 1, according to the invention, a heat adjustment device 10 of the invention comprises a heat conductive substrate 11 and a plurality of thermoelectric chips 12, 13, 14, 15 and 16. The heat conductive substrate 11 has a support surface 111. Each of the thermoelectric chips has a first surface and a second surface, wherein the second surfaces of the thermoelectric chips are fixed to the support surface of the heat conductive substrate. Now taking the thermoelectric chip 12 as an example for illustration, the thermoelectric chip 12 has a first surface 121 and a second surface 122. The second surface 122 of the thermoelectric chip 12 is fixed to the support surface 111 of the heat conductive substrate 11. The thermoelectric chips can be fixed to the support surface 111 of the heat conductive substrate 11 by a heat conducting glue. The heat conductive substrate 11 may be a metal plate for the benefit of conducting heat.

In this embodiment, the thermoelectric chips 12, 13, 14, 15 and 16 are electrically connected in parallel. In other words, each thermoelectric chip has two power input ends, one being positive and the other being negative. In this embodiment, the positive power input end and the negative power input end of each thermoelectric chip are connected in parallel and connected to a power supply 21. The power supply 21 may be a battery for the convenience of carry. The heat adjustment device of the invention is not limited to have the thermoelectric chips connected in parallel, and the thermoelectric chips may also be connected in serial, or in a mixed manner of serial and parallel connection.

The heat adjustment device 10 of the invention further comprises a temperature control circuit 22. The temperature control circuit 22 is connected between the power supply 21 and the thermoelectric chips to adjust the temperature variation of the second surfaces of the thermoelectric chips. Particularly, the temperature control circuit 22 adjusts the power supplied to the thermoelectric chips so as to adjust the temperature variation of the second surfaces of the thermoelectric chips. That is, when a larger voltage or current is supplied to the thermoelectric chips, the temperature variation of the thermoelectric chips is greater. If the second surface is the end producing heat, the extent of temperature increasing of the second surface will be increased so that more heat will be produced; if the second surface is a cooling end, the extent of temperature decreasing of the second surface will be increased.

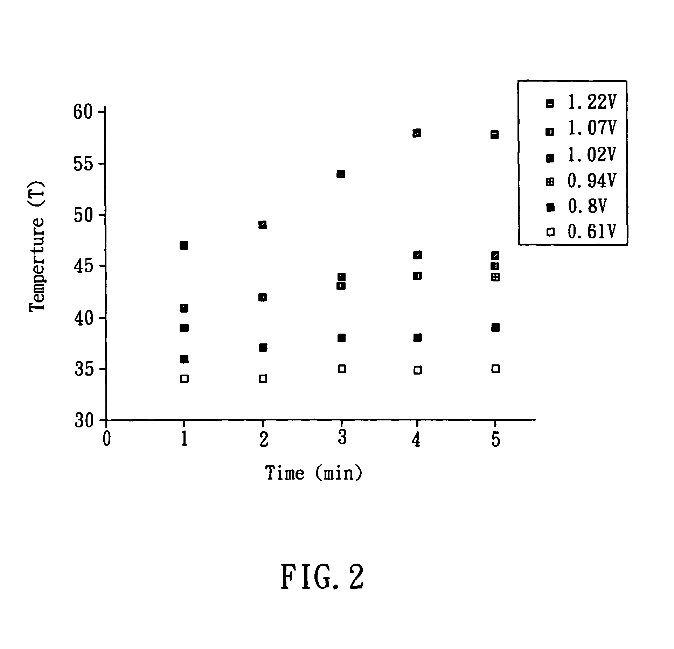

After the power supply is provided to the thermoelectric chips, a temperature variation will be produced on the second surfaces of the thermoelectric chips, and conducted to the heat conductive substrate 11, so that the heat conductive substrate 11 has a corresponding temperature variation. Fig. 2 shows the corresponding temperature variations of the heat conductive substrate 11 for different input voltages. In the environment of room temperature 25°C, when the input voltage to the thermoelectric chips is low(for example, of 0.61V), the temperature increasing of the heat conductive substrate 11 is not obvious, about below 35°C. When the input voltage is elevated gradually(for example, of 1.22V), the extent of temperature increasing of the heat conductive substrate 11 is greater, and the maintained temperature is also higher, up to about 60°C. For this embodiment, when the input voltage is 1.07V, the extent of temperature increasing of the heat conductive substrate 11 and the maintained temperature shall preferably be about 40-45°C. However, the thermoelectric chips of different model will produce different extents of temperature increasing and maintained temperatures.

According to the heat adjustment device of the invention, after the temperature variation is conducted by the thermoelectric chips to the heat conductive substrate, a corresponding temperature variation is produced on the heat conductive substrate so that the area of the temperature variation is increased and the temperature is uniformly distributed on the heat conductive substrate. The heat adjustment device of the invention can be mounted in clothes. When the temperature of the second surfaces of thermoelectric chips is increased and the heat is conducted to the heat conductive substrate, the clothes can produce heat to keep warm.

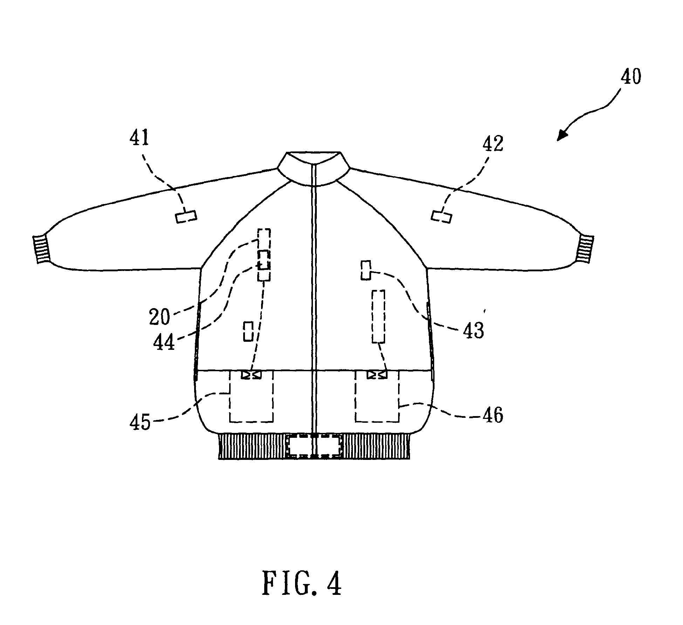

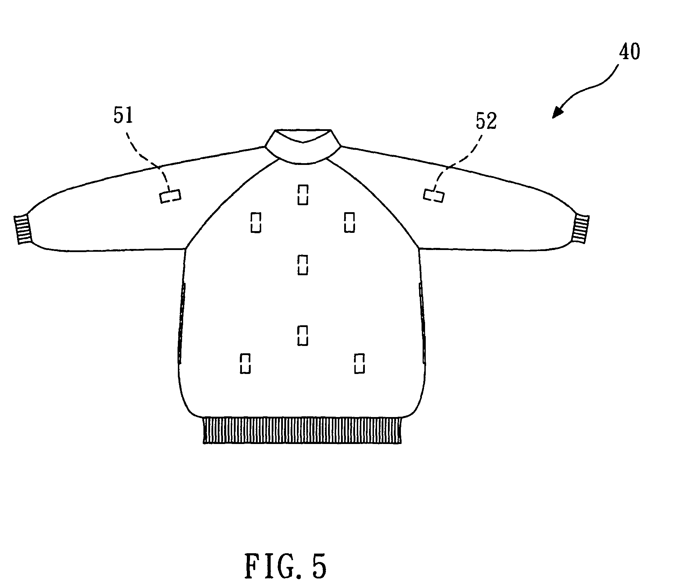

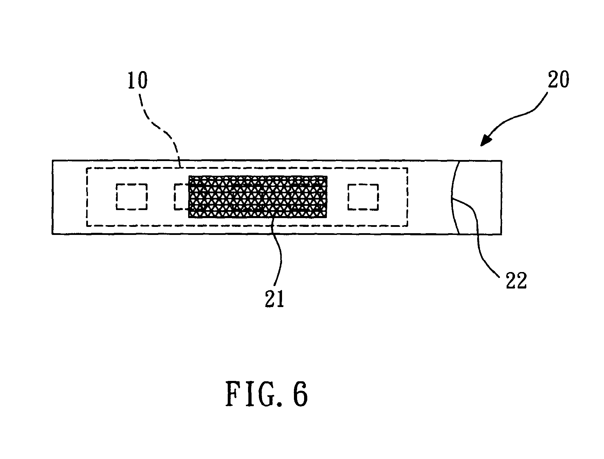

Referring to Fig. 4 and Fig. 5, they show a jacket 40 with a plurality of magic sticks 41, 42, 43, 44, 51 and 52. The magic stickers 41, 42, 51 and 52 are fixed on the sleeves of the jacket 40, and the magic stickers 43 and 44 are fixed on the body portion of the jacket 40. The magic stickers are utilized to engage with another magic sticker 21 of a packet 20 as shown in Fig. 6. The packet 20 is utilized to contain the heat adjustment device 10 of the invention. The packet 20 comprises a magic sticker 21 and an open 22. The heat adjustment device 10 of the invention is mounted in the packet 20 through the open 22. The packet 20 can be attacked to the jacket 40 by engaging the magic sticker 21 of the packet 20 and the magic sticker 44 of the jacket 40. Furthermore, the jacket 40 comprises two battery bags 45, 46 for containing battery providing power to the heat adjustment device 10 of the invention. Therefore, by utilizing the heat adjustment device 10 of the invention, the jacket 40 can produce heat to keep warm.

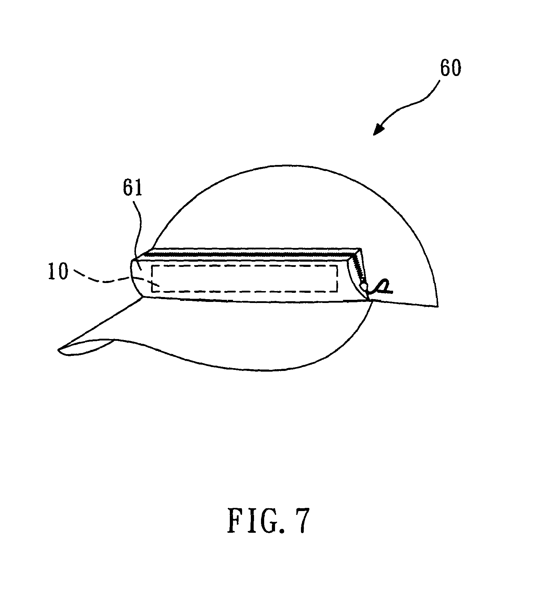

Referring to Fig. 7, it shows a hat 60 with a bag 61. The bag 61 is utilized to contain the heat adjustment device 10 of the invention. The heat adjustment device 10 in the hat 60 can be utilized to produce heat or dissipate heat. Therefore, people who put on the hat can feel warm or cool.

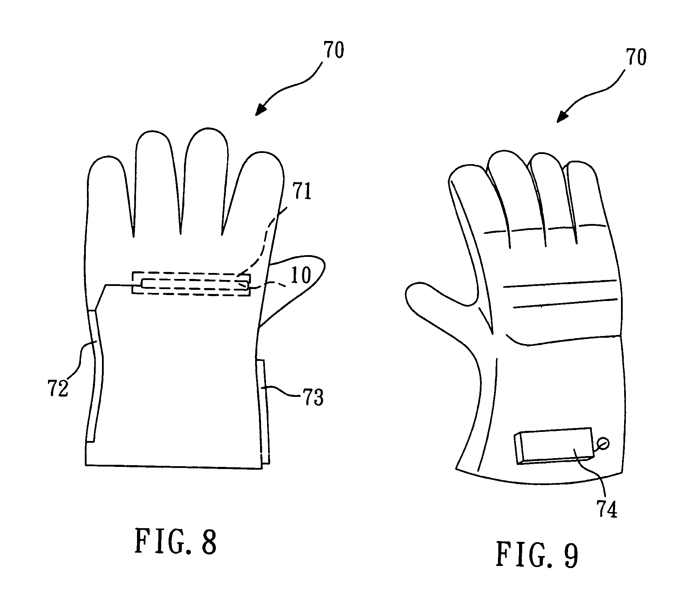

Referring to Fig. 8 and Fig. 9, they show a glove 70 with at least one packet 71 and a bag 74. The packet 71 is disposed on the inside of the glove 70, and the bag 74 is disposed on the outside of the glove 70. The packet 71 can be utilized to contain the heat adjustment device 10 of the invention. The heat adjustment device 10 in the glove 70 can be utilized to produce heat, and the glove 70 can produce heat to keep warm. The bag 74 is used to contain battery providing power to the heat adjustment device 10 of the invention. Furthermore, the glove 70 comprises a fixed element 72 and a zipper 73. The fixed element 72 is a cylindrical cloth for fixing the wire between the heat adjustment device 10 and the battery. The zipper 73 is used for installing or unloading the heat adjustment device 10.

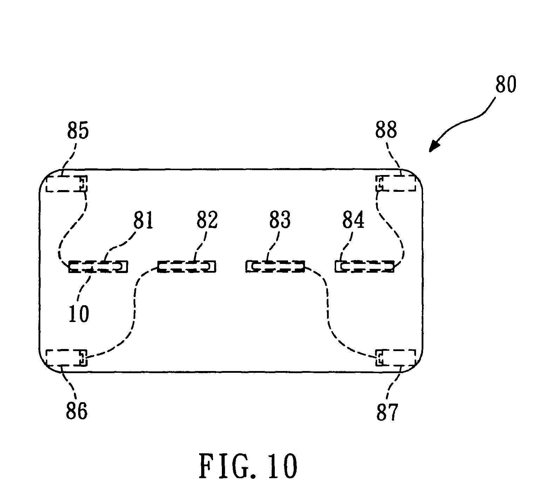

Referring to Fig. 10, it shows a mattress 80 with a plurality of packets 81, 82, 83, and 84. The packets 81, 82, 83, and 84 are fixed on the mattress 80. The packets 81, 82, 83, and 84 are utilized to contain the heat adjustment device 10 of the invention. The packets 81, 82, 83, and 84 can be attacked to the mattress 80. Furthermore, the mattress 80 comprises a plurality of battery bags 85, 86, 87, and 88 for containing batteries providing power to the heat adjustment device 10 of the invention. Therefore, by utilizing the heat adjustment device 10 of the invention, the mattress 80 can produce heat to keep warm.

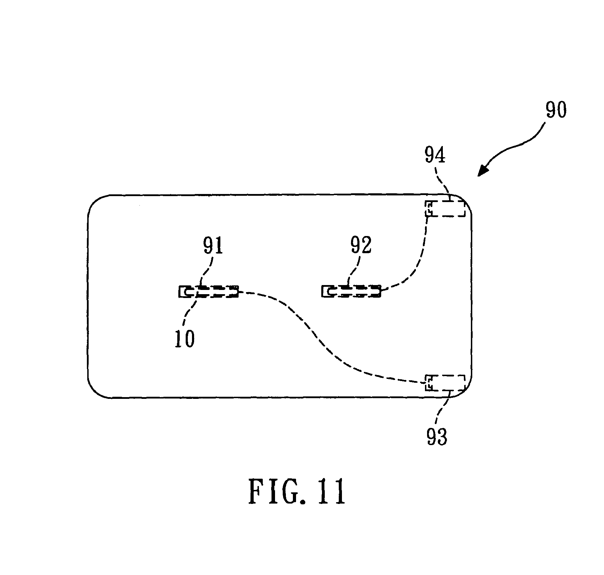

Referring to Fig. 11, it shows a bedclothes 90 with a plurality of packets 91 and 92. The packets 91 and 92 are fixed on the bedclothes 90. The packets 91 and 92 are utilized to contain the heat adjustment device 10 of the invention. The packets 91 and 92 can be attacked to the bedclothes 90. Furthermore, the bedclothes 90 comprises a plurality of battery bags 93 and 94 for containing batteries providing power to the heat adjustment device 10 of the invention. Therefore, by utilizing the heat adjustment device 10 of the invention, the bedclothes 90 can produce heat to keep warm.

While an embodiment of the present invention has been illustrated and described, various modifications and improvements can be made by those skilled in the art. The embodiment of the present invention is therefore described in an illustrative, but not restrictive, sense. It is intended that the present invention may not be limited to the particular forms as illustrated, and that all modifications which maintain the spirit and scope of the present invention are within the scope as defined in the appended claims.

## Claims

1. A heat adjustment device, comprising:
a heat conductive substrate having a support surface; and
a plurality of thermoelectric chips, each of thermoelectric chips having a first surface and a second surface, wherein the second surfaces of the thermoelectric chips are fixed to the support surface of the heat conductive substrate, and the thermoelectric chips are electrically connected for providing power to the thermoelectric chips so that the second surfaces of the thermoelectric chips have a temperature variation conducted to the heat conductive substrate.

2. The heat adjustment device according to Claim 1, wherein each of the thermoelectric chips has at least one power input end, and the thermoelectric chips are electrically connected in parallel.

3. The heat adjustment device according to Claim 1, wherein each of the thermoelectric chips has at least one power input end, and the thermoelectric chips are electrically connected in serial.

4. The heat adjustment device according to Claim 1, further comprising a power supply for providing the power required by the thermoelectric chips.

5. The heat adjustment device according to Claim 4, wherein the power supply is a battery.

6. The heat adjustment device according to Claim 1, wherein after the thermoelectric chips are driven by the power, the second surfaces give a temperature variation of temperature increasing.

7. The heat adjustment device according to Claim 1, wherein after the thermoelectric chips are driven by the power, the second surfaces give a temperature variation of temperature decreasing.

8. The heat adjustment device according to Claim 1, further comprising a temperature control circuit to adjust the temperature variation of the second surfaces of the thermoelectric chips.

9. The heat adjustment device according to Claim 8, wherein the temperature control circuit adjusts the power supplied to the thermoelectric chips so as to adjust the temperature variation of the second surfaces of the thermoelectric chips.

10. The heat adjustment device according to Claim 1, wherein the thermoelectric chips are fixed to the support surface of the heat conductive substrate by a heat conducting glue.

11. The heat adjustment device according to Claim 1, wherein the heat conductive substrate is a metal plate.

12. The heat adjustment device according to Claim 1, further comprising a packet for containing the heat conductive substrate and thermoelectric chips.

13. The heat adjustment device according to Claim 12, wherein the packet comprises a magic sticker.
